# EUROPEAN PATENT SPECIFICATION

(11) **EP 2 419 094 B1**
(45) Date of publication and mention of the grant of the patent: **08.06.2016**
(21) Application number: 10719072.0
(22) Date of filing: 15.04.2010
(51) Int. Cl.: A61K 31/4425, A61P 43/00

(54) **Compounds for the treatment of mitochondrial diseases**
Verbindungen zur Behandlung von mitochondrialen Erkrankungen
Composés pour le traitement de maladies mitochondriales

(30) Priority: 17.04.2009 EP 09290288
(43) Date of publication of application: 22.02.2012
(73) Proprietor: Centre National de la Recherche Scientifique, 75016 Paris (FR); Institut Curie, 75248 Paris Cedex 05 (FR); UNIVERSITY OF PITTSBURGH, Pittsburgh PA 15260 (US)
(72) Inventor: BLONDEL, Marc, F-29250 Saint Pol de Léon (FR); COUPLAN, Elodie, F-29200 Brest (FR); DI RAGO, Jean-Paul, F-33480 Sainte-Hélène (FR); DAUZONNE, Daniel, F-75011 Paris (FR); PALLADINO, Michael, Pittsburgh, Pennsylvania 15260 (US); CELOTTO, Alicia, Pittsburgh, Pennsylvania 15260 (US)
(74) Representative: Gevers & Orès
(86) International application number: PCT/IB2010/001006
(87) International publication number: WO 2010/119344

(56) References cited:
- EP-A- 1 550 442
- WO-A-95/19170
- WO-A-2007/002497
- WO-A-2007/095630
- WO-A1-01/29034
- US-A- 2 745 826
- ROTA M T ET AL: "Reduction of oral acetaldehyde levels using a controlled-release chlorhexidine chip as a prevention strategy against upper digestive tract cancer." MEDICAL HYPOTHESES JUN 2003 LNKD- PUBMED:12699713, vol. 60, no. 6, June 2003 (2003-06), pages 856-858, XP009136018 ISSN: 0306-9877
- YIP KENNETH W ET AL: "Benzethonium chloride: a novel anticancer agent identified by using a cell-based small-molecule screen." CLINICAL CANCER RESEARCH : AN OFFICIAL JOURNAL OF THE AMERICAN ASSOCIATION FOR CANCER RESEARCH 15 SEP 2006 LNKD- PUBMED:17000693, vol. 12, no. 18, 15 September 2006 (2006-09-15), pages 5557-5569, XP009136017 ISSN: 1078-0432
- DAUZONNE D ET AL: "Synthesis and in vitro cytotoxicity of a series of 3-aminoflavones" EUROPEAN JOURNAL OF MEDICINAL CHEMISTRY, vol. 32, no. 1, 1 January 1997 (1997-01-01), pages 71-82, XP004034181 ISSN: 0223-5234 cited in the application
- DAUZONNE D ET AL: "A convenient synthesis of 3-chloro-3,4-dihydro-4-hydroxy-3-nitro-2-p henyl-2H-1-benzopyrans" SYNTHESIS, vol. 1990, no. 1, 1990, pages 66-70, XP009121832 ISSN: 0039-7881 cited in the application
- DAUZONNE, DANIEL ET AL: "Synthesis of 2-aryl-3-nitro-4H-1-benzopyran-4-ones" SYNTHESIS, vol. 1992, no. 7, July 1992 (1992-07), pages 677-680, XP009121862 ISSN: 0039-7881
- GONZALEZ DE PEREDO A ET AL: "Synthesis and biological evaluation of flavanones and flavones related to podophyllotoxin." CHEMICAL & PHARMACEUTICAL BULLETIN, vol. 46, no. 1, January 1998 (1998-01), pages 79-83, XP009121834 ISSN: 0009-2363 cited in the application
- BAUVOIS B ET AL: "Synthesis and biological evaluation of novel flavone-8-acetic acid derivatives as reversible inhibitors of aminopeptidase N/CD13" JOURNAL OF MEDICINAL CHEMISTRY, vol. 46, no. 18, 28 August 2003 (2003-08-28), pages 3900-3913, XP001181149 ISSN: 0022-2623 cited in the application
- DAUZONNE D ET AL: "Synthesis of the 3-aminoflavone-8-acetic acid" TETRAHEDRON LETTERS, vol. 36, no. 11, 13 March 1995 (1995-03-13), pages 1845-1848, XP004028502 ISSN: 0040-4039

## Description

The present invention relates to sodium pyrithione for use in preventing and/ or treating mitochondrial diseases and/or mitochondrial alterations and mitochondrial disorders observed during the progress of neurodegenerative diseases.

The present disclosure relates to the isolation and development of drugs to treat mitochondrial pathologies involving a deficiency in ATP production via the oxidative phosphorylation pathway, such as NARP syndrome.

NARP (Neuropathy, Ataxia and Retinitis Pigmentosa) is a maternally transmitted hereditary syndrome characterized by retarded development, and accompanied by retinitis pigmentosa (RP), dementia, ataxia, proximal neurological muscle weakness and sensory neuropathies (Schon et al., J. Bioenerg. Biomembr., 1994, 26, 291-299; Graeber, M.B. and Müller, U., J. Neurol. Sci., 1998, 153, 251-263, for review). This disease is in general a pathology which occurs in children, but it has also been reported in rarer cases in adults. The clinical manifestations are varied and can take more or less severe forms. Thus, the ophthalmic manifestations can range from a simple "salt and pepper" changing of the retina to severe RP, accompanied by maculopathy. Similarly, there is a broad spectrum of neurological manifestations, which ranges from simple migraines to severe dementia and to "Leigh's disease" (subacute necrotising encephalomyelopathy; Ortiz et al., Arch., Ophtalmol., 1993, 111, 1525-1530). Many retinitis pigmentosa-related syndromes exist, such as Usher's syndrome in which both the sight and the hearing are affected, or else macular dystrophy, also called inverse RP.

In 1990, Holt et al. (Am. J. Hum. Genet., 46, 428-433) described for the first time the presence of the T8993G mutation in the mitochondrial DNA of patients showing NARP syndrome/Leigh's disease. It was subsequently postulated by Tatuch and Robinson (Biochem. Biophys. Res. Commun., 1993, 192, 124-128) that this mutation -occurring in the ATP6 subunit- resulted in a reduction in ATP synthesis by impairing the mitochondrial ATP synthase complex. This mutation is thought to be responsible for an ATP synthase assembly/stability defect (Nijtmans et al., J. Biol. Chem., 2001, 276, 6755-6762). Other *ATP6* gene mutations have also been detected, in association with NARP syndrome/Leigh's disease; T8993C, T9176G, T9176C, T8851C, T9185C and T9191C (Schon et al., Cell & Dev. Biol., 2001, 12, 441-448 and Kucharczyk et al., Biochimica et Biophysica Acta, 2009, 1793, 186-199). In addition, a T9101C mutation has been involved in the LHON (*Leber*'*s Hereditary Optic Neuropathy*) syndrome, another mitochondrial syndrome (Kucharczyk et al., Biochimica et Biophysica Acta, 2009, 1793, 186-199). Simple point mutations are therefore responsible for these syndromes, which have many more or less serious forms. The great diversity of the pathological manifestations is attributed to the heteroplasmic nature of this mutation in patients, i.e. the coexistence of mutated and wild-type mitochondrial DNA molecules in the cells or tissues. The mutated mitochondrial DNA load is closely correlated with the seriousness of the symptoms observed (Uziel et al., J. Neurol. Neurosurg. Psychiatry, 1997, 63, 16-22; Carelli et al., Arch. Neurol., 2002, 59, 264-270).

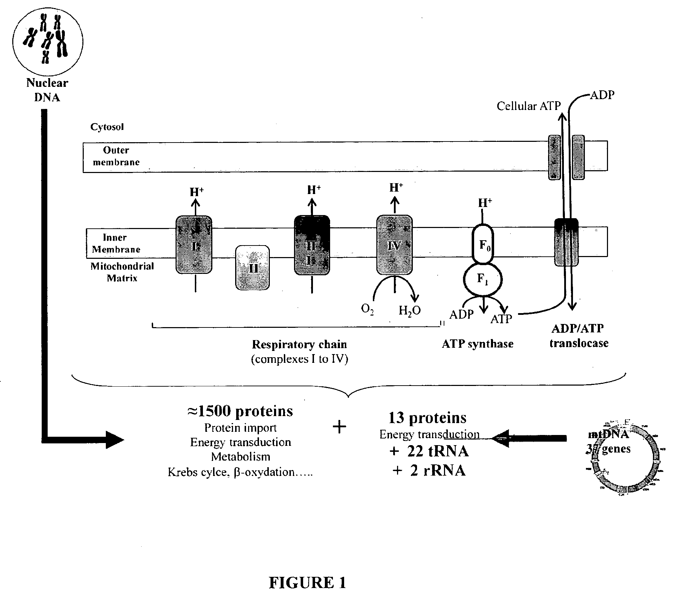

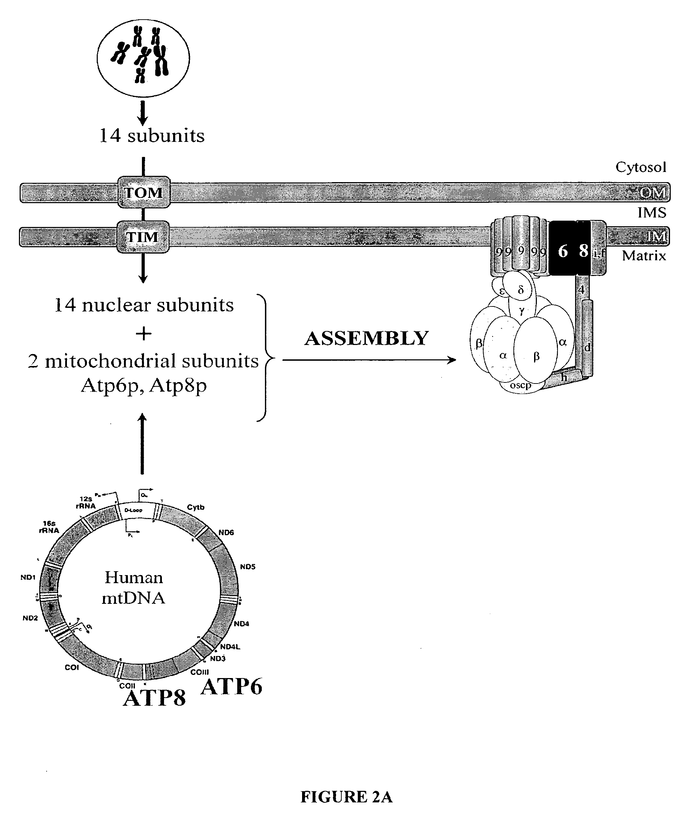

The ATP synthase complex, which is the target of the T8993G mutation (and of the other mutations mentioned above), is located in the inner mitochondrial membrane (Figures 1 and 2A). It catalyzes the last steps of oxidative phosphorylation, a process which allows cells to extract the chemical energy of metabolites and to store this energy in ATP molecules. In order to synthesize ATP, the ATP synthase complex uses the electrochemical proton gradient on either side of the inner membrane, generated by other complexes located in this membrane, the respiratory complexes (Figure 1). The latter transfer to oxygen the reducing equivalents of the substrates that are oxidized in the mitochondrion. These transfers are coupled to proton transports (hydrogen ions, H⁺) across the inner membrane, from the inside (the mitochondrial matrix) into the space between the outer and inner membranes (intermembrane space) of the organelle. The result is a proton concentration that is higher at the outer periphery of the inner membrane than at its inner periphery. The membrane domain Fₒ (Figure 1) of ATP synthase enables a channeled return of the protons into the mitochondrial matrix. This transport is coupled to ATP synthesis in the catalytic domain F₁ of ATP synthase located outside the membrane, in the mitochondrial matrix. ATP synthase operates like a rotary turbine: the passage of protons in Fₒ is coupled to the rotation of a subcomplex (the rotor) of the enzyme. This rotation results in conformational changes in F₁ which promote the synthesis of ATP from ADP and inorganic phosphate (Boyer P.D., Annu, Rev., Biochem., 1997, 66, 717-747). The neosynthesized ATP molecules can, via a specific transporter located in the inner membrane (ADP/ATP translocase), leave the mitochondrial compartment so as to supply the entire cell with energy. ATP synthase comprises about twenty different protein subunits for a mass of approximately 600 KDa. In humans, two ATP synthase subunits (Atp6p and Atp8p, Figure 2A) are encoded by the mitochondrial genome, all the other subunits being encoded by nuclear genes. The subunits of nuclear origin are synthesized in the cytosol and then imported into the mitochondrion, whereas the Atp6p and Atp8p subunits encoded by the mitochondrial genome are actually synthesized inside the mitochondrion (Figure 2A).

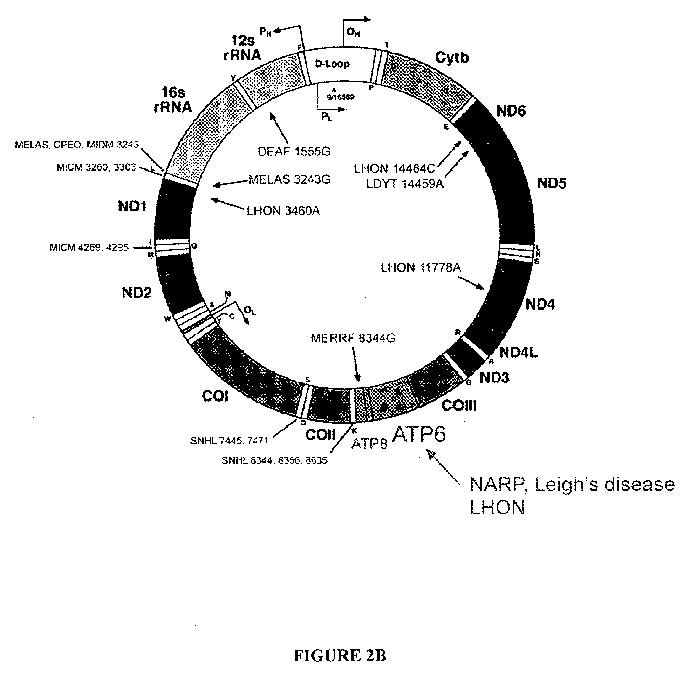

The T8993G mutation associated with NARP syndrome is located within the mitochondrial *ATP6* gene (Figure 2B). The latter encodes ATP synthase subunit 6 (Atp6p) which is essential for proton transport across Fₒ. The T8993G mutation results in the replacement, with arginine, of a leucine residue conserved in all the known sequences of Atp6p, from bacteria to humans. This leucine residue is in an Atp6p region presumed to be transmembrane and essential for ATP synthase proton translocation activity. Studies carried out in the *Escherischia coli* bacterium or with NARP cybrids (human cells in which the mitochondria are enriched, up to 100%, in T8993G alleles) indicate that the T8993G mutation clearly affects the functioning of the ATP synthase proton channel and that this defect is the primary cause of the disease (Schon et al., Cell & Dev. Biol., 2001, 12, 441-448; Nijtmans et al., J. Biol. Chem., 2001, 276, 6755-6762).

WO 2007/095630 A2 discloses ubiquinone analogues for the treatment of mitochondrial diseases. EP1550442 A1 discloses L-arginine for treating the expression of clinical symptoms in a disease caused by mitochondrial dysfunction. WO9519170 discloses riluzole for the treatment of mitochondrial diseases.

However, there is currently no effective medicament for the treatment of mitochondrial disorders induced by F₁F₀-ATP synthase dysfunctions.

A cellular model for the NARP syndrome has recently been developed consisting in yeast strains carrying within their mitochondrial genome, the equivalent of mitochondrial *ATP6* gene mutations responsible for NARP syndrome in humans (see the International PCT Application WO 2007/125225).

These yeast mutants make it possible to identify molecules capable of correcting the effects of the mutation by restoring either ATP synthase function, or sufficient production of ATP in the mitochondria, via a pathway other than that of oxidative phosphorylation.

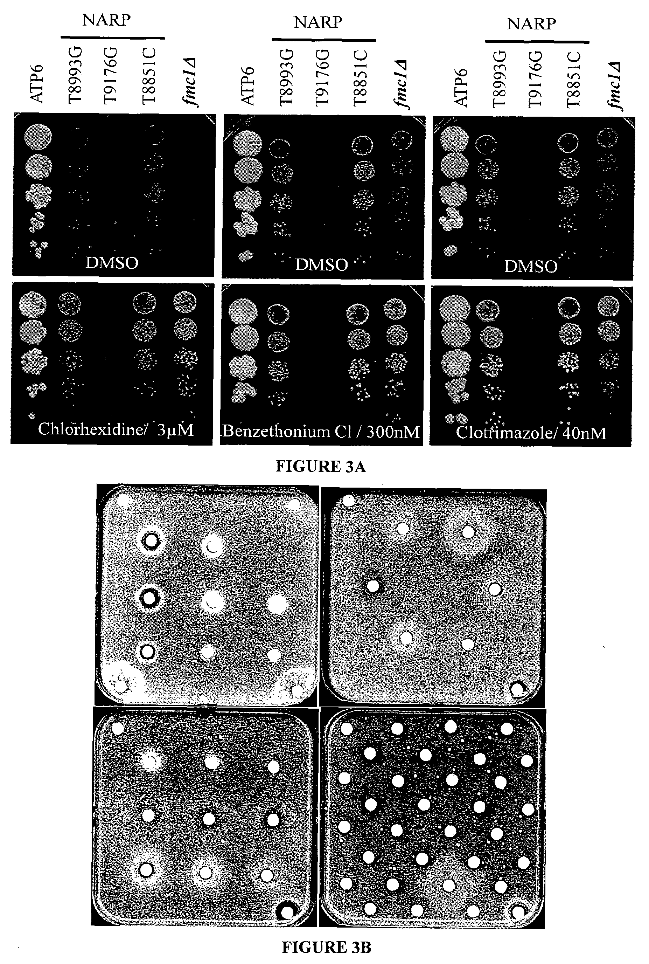

The disclosure concerns compounds which have been selected for their ability to restore respiratory growth of the yeast *ATP6* mutant (Figures 3A and 3B).

An object of the present disclosure concerns the use of compounds having the general formula (I): 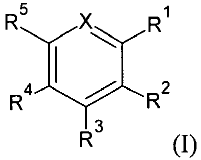 wherein
- **X** is a carbon atom or a nitrogen atom, both atoms being optionally substituted by an oxygen atom;
- **R¹** is a hydrogen atom, a halogen atom or a sulphur atom;
- **R²** is a hydrogen atom or
   **R¹** and **R²** taken together with the carbon atoms to which they are attached may form the following cycle 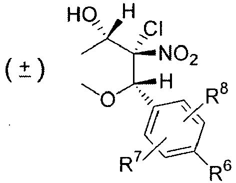 with the proviso that when **R¹** and **R²** form the above defined cycle, **X** is a carbon atom;
- **R³** is a hydrogen atom; a halogen atom; an alkyl radical containing 1 to 6 carbon atoms, preferably 1 to 3 carbon atoms, optionally interrupted by one oxygen atom or one ester function or an alkenylene radical containing 2 to 6 carbon atoms, preferably 2 to 3 carbon atoms,
   with the proviso that when **R¹** and **R²** do not correspond to the above defined cycle, **R³** is a hydrogen atom;
- **R⁴** is:
   - a hydrogen atom,
   - an alkyl radical containing 1 to 6 carbon atoms, preferably 1 to 3 carbon atoms, optionally interrupted by one oxygen atom,
   - a linear chain containing between 5 and 15 carbon atoms and between 1 and 10 nitrogen atoms,
   said chain optionally contains 1 to 5 oxygen atoms and is optionally interrupted by a benzyl group optionally substituted by one halogen atom; carbon and/or nitrogen atoms of said chain being optionally substituted by 1 or 2 alkyl radicals containing 1 to 3 carbon atoms, preferably a methyl radical, and carbon atoms of said chain being optionally substituted with =NH function;
- **R⁵** is a hydrogen atom, a halogen atom, or a group 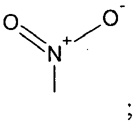
   **R⁴** and **R⁵** taken together with the carbon atoms to which they are attached may form the following cycle 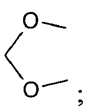
- **R⁶**, **R⁷** and **R⁸**, which may be identical or different, are:
   - a hydrogen atom;
   - a halogen atom;
   - an alkyl radical containing 1 to 6 carbon atoms, preferably 1 to 3 carbon atoms, optionally interrupted by one oxygen atom or one ester function and optionally containing a benzyl group;
   - a group -NH₂;
   - a group 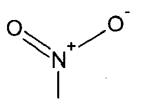
      **R⁶** and **R⁸** taken together with the carbon atoms to which they are attached may form a benzyl group;
      with the proviso that
      when R¹ and R² correspond to the above defined cycle, R⁴ = R⁵ = R⁷ = R⁸ = H and R⁶ = Cl, R³ is not -O-CH₃ or
      when R¹ and R² correspond to the above defined cycle, R³ = R⁵ = R⁴ = R⁸ = H, and R⁷ is in ortho-position of R⁶, R⁶ and R⁷ are not simultaneously -O-CH₃,
      or a pharmaceutical acceptable salt
      for the preparation of a drug for the prevention and/or the treatment of disorders or diseases related to an insufficiency or a lack of ATP synthesis by F₁F₀-ATP synthase or related to an excessive accumulation of reactive oxygen species (ROS) chosen amongst mitochondrial diseases, normal or physiological ageing and neurodegenerative diseases.

By "halogen atom" is intended to mean fluorine, chlorine, bromine or iodine.

By "alkyl radical" containing 1 to 6 carbon atoms, is intended to mean a saturated, linear or branched, chain of 1 to 6 carbon atoms, such as methyl, ethyl, propyl, isopropyl, butyl, pentyl, hexyl.

By "alkenylene radical containing 2 to 6 carbon atoms", is intended to mean an unsaturated, linear or branched, chain of 2 to 6 carbon atoms containing one carbon-to-carbon double bond.

Examples of alkyl radicals containing 1 to 6 carbon atoms interrupted by one oxygen atom are of the general formula -(CH₂)ₙ-O-(CH₂)_{n'}-CH₃, n and n' being two integers comprised between 0 and 6 such as n + n' ≤6; an example of such radical is -O-CH₃.

Example of alkyl radicals containing 1 to 6 carbon atoms, preferably 1 to 3 carbon atoms, optionally interrupted by one oxygen atom or one ester function and optionally containing a benzyl group are: -O-CH₃, -O-CH₂-C₆H₆, -CO-O-CH₃, and -CO-O-CH₂-C₆H₆.

A non-limitating example of linear chain containing between 5 and 15 carbon atoms and between 1 and 10 nitrogen atoms; said chain being optionally interrupted by a benzyl group optionally substituted by one halogen atom; carbon atoms of said chain being optionally substituted with =NH function is the following: - NH-C(NH)-NH-C(NH)-NH-(CH₂)₆-NH-C(NH)-NH-C(NH)-NH-C₆H₄-Cl.

A non-limitating example of linear chain containing between 5 and 15 carbon atoms and between 1 and 10 nitrogen atoms, said chain optionally containing 1 to 5 oxygen atoms and being optionally interrupted by a benzyl group; carbon and/or nitrogen atoms of said chain being optionally substituted by 1 or 2 alkyl radicals containing 1 to 3 carbon atoms, preferably a methyl radical; is the following:-CH₂-NH₂-(CH₂)₂-O-(CH₂)₂-O-C₆H₄-C(CH₃)₂-CH₂-C(CH₃)₂-CH₃.

In a first mode of carrying out the disclosure compounds of formula (I) are such as R¹ and R² do not correspond to the above-defined cycle; then preferred compounds are selected in the following compounds :
Compound of the following formula A: chlorhexidine: for the compound of formula (I), wherein X = C, R¹ = Cl, R² = R3 = R⁵ = H and R = -NH-C(NH)-NH-C(NH)-NH-(CH₂)₆-NH-C(NH)-NH-C(NH)-NH-C₆H₆-Cl; 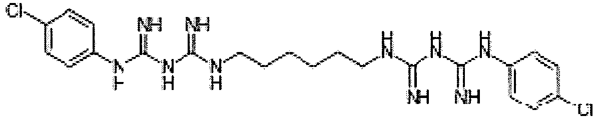
Compound of the following formula B: benzethonium chloride: for the compound of formula (I), wherein X = C, R¹ = R² = R³ =R⁵ = H and R⁴ = -CH₂-N⁺(CH₃)(CH₃)-(CH₂)₂-O-(CH₂)₂-O-C₆H₆-C(CH₃)₂-CH₂-C(CH₃)₂-CH₃.

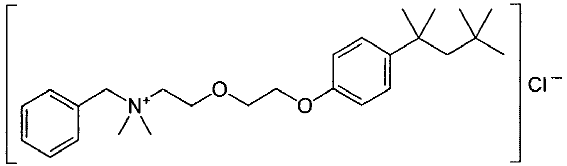

1 In a first mode of carrying out the invention, compound of formula (I) is such as R and R do not correspond to the above-defined cycle, then preferred compound is: Compound of the following formula D: sodium pyrithione: for the compound of formula (I), wherein X = N+-O , R¹ = S and R² = R³ = R⁴ = R⁵ = H.

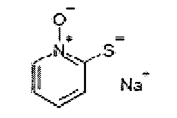

Chlorhexidine is known to have antiseptic properties, it shows also bactericidal properties, it kills both gram-positive and gram-negative bacteria.

Benzethonium chloride is a synthetic quaternary ammonium salt with surfactant, antiseptic and anti infective properties.

Sodium pyrithione (CAS Registry number: 15922-78-8 ; 3811-73-2) is a large spectrum antimicrobial agent; it inhibits the growth of fungi, yeast, mold and bacteria.

Those three compounds are commercially available; their syntheses are reported in the literature: chlorexidine may be prepared according to US Patent 2,684,924; benzethonium chloride may be prepared according to US Patents 2,115,250, 2,170,111 and 2,229,024; clotrimazole may be prepared according to South African Patents 68 05,392 and 69 00,039 (Bayer) and sodium pyrithione may be prepared according to Shaw et al. J. Amer. Chem Soc. 72, 4362 (1950) or to US Patent 2,745,826.

In another mode of carrying out the disclosure compounds of formula (I) are such as R¹ and R² form the above-defined cycle; the preferred compounds are those defined by the general formula (Ia): 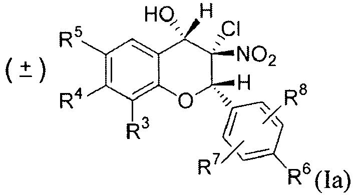
wherein **R³, R⁵, R⁶, R⁷** and **R⁸** are defined as above;
   - **R⁴** is:
      - a hydrogen atom;
      - an alkyl radical containing 1 to 6 carbon atoms, preferably 1 to 3 carbon atoms, optionally interrupted by one oxygen atom; or
      **R⁴** and **R⁵** taken together with the carbon atoms to which they are attached may form the cycle 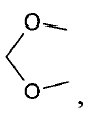 or a pharmaceutical acceptable salt of compounds of general formula (Ia).

According to a particular object, the disclosure relates to compounds of general formula (Ia) as drug, particularly, as active agents.

The following compounds of formula (Ia) are preferred:
Compound of Formula 1. (ICC005-L-001-A11) 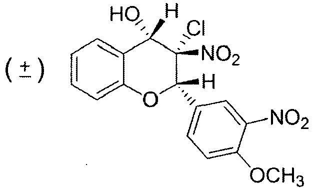
Compound of Formula 2. (ICC005-M204-C05) 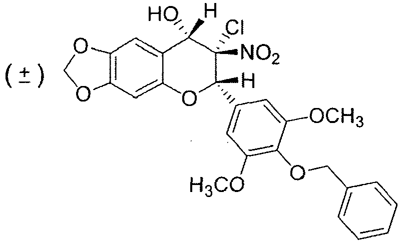
Compound of Formula 3. (ICC005-L006-D11) 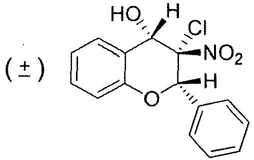
Compound of Formula 4. (ICC005-L028-D02) 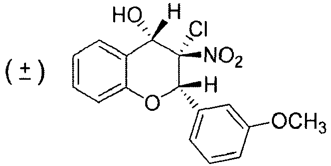
Compound of Formula 5. (ICC005-L025-A02) 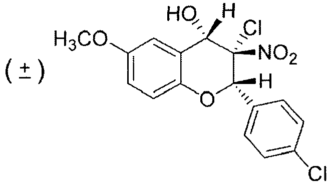
Compound of Formula 6. (ICC005-L023-B02) 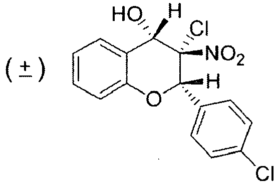
Compound of Formula 7. (ICC005-L040-D06) 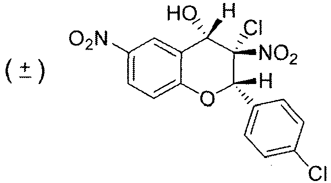
Compound of Formula 8. (ICC005-L134-H07) 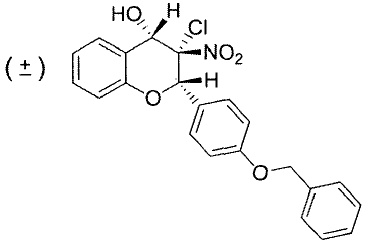
Compound of Formula 9. (ICC005-L022-B05) 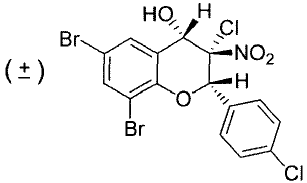
Compound of Formula 10. (ICC005-L040-E11) 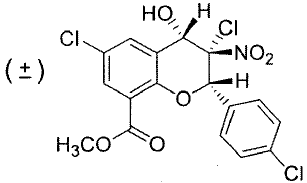
Compound of Formula 11. (ICC005-L037-H11) 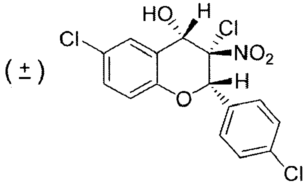
Compound of Formula 12. (ICC005-L037-G07) 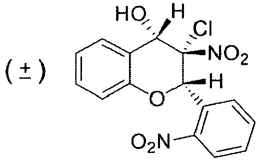
Compound of Formula 13. (ICC005-L036-A07) 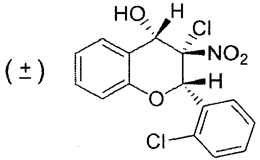
Compound of Formula 14. (ICC005-L024-E06) 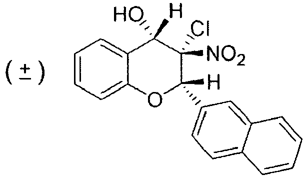
Compound of Formula 15. (ICC005-L032-H02) 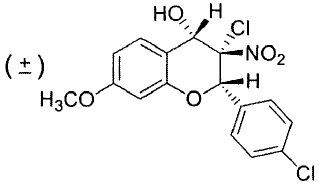
Compound of Formula 16. (ICC005-L034-E11) 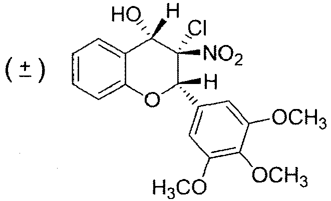
Compound of Formula 17. (ICC005-L033-E02) 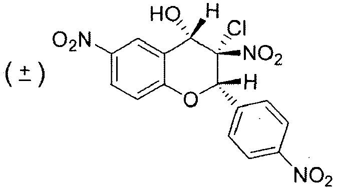
Compound of Formula 18. (ICC005-L018-C11) 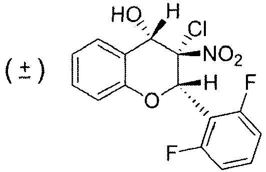
Compound of Formula 19. (ICC005-L028-E06) 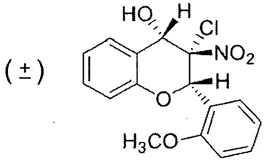
Compound of Formula 20. (ICC005-L018-E05) 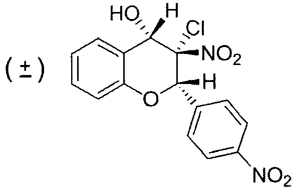
Compound of Formula 21. (ICC005-L002-D06) 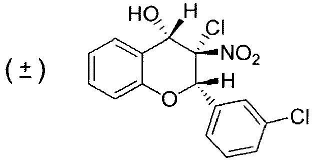
Compound of Formula 22. (ICC005-L002-F04) 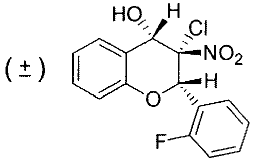
Compound of Formula 23. (ICC005-L002-C03) 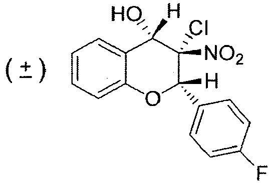
Compound of Formula 24. (ICC005-L002-G02) 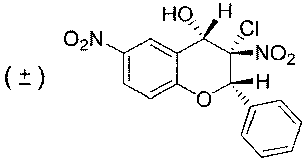
Compound of Formula 25. (ICC005-L019-B10) 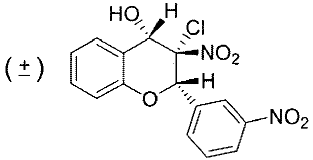
Compound of Formula 26. (ICC005-L001-B11) 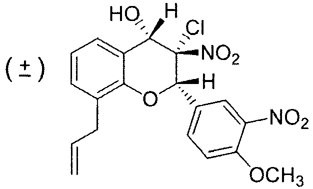
-13-Compound of Formula 27. (ICC005-LO15-E03) 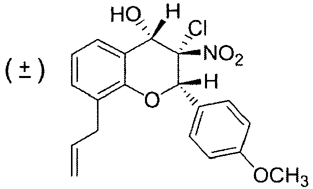
Compound of Formula 28. (ICC005-M204-C06) 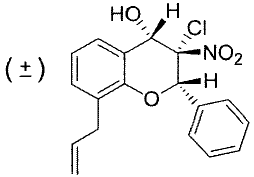
Compound of Formula 29. (ICC005-L145-E05) 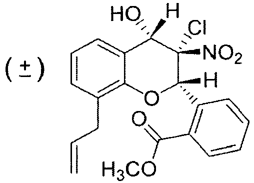
Compound of Formula 30. (ICC005-L145-E11) 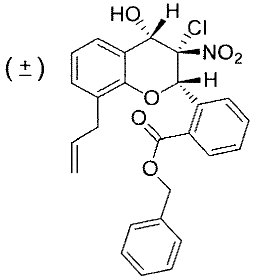
Compound of Formula 31. (ICC005-L145-H02) 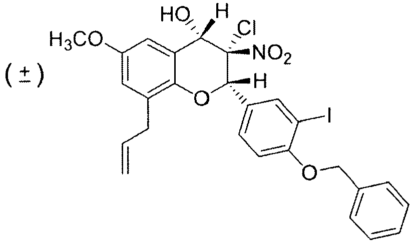
Compound of Formula 32. (ICC005-L145-H06) 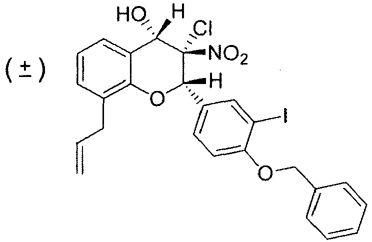
Compound of Formula 33. (ICC005-L046-F07) 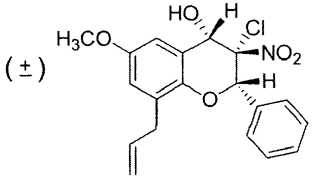
Compound of Formula 34. (ICC005-L044-H06) 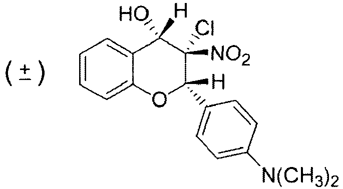
Compound of Formula 35. (ICC005-L046-C11) 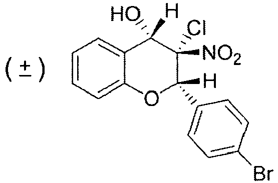
Compound of Formula 36. (ICC005-L045-E06)

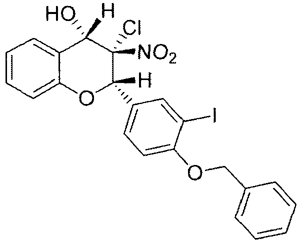

The synthesis of compounds of general formula (Ia) is described below.

Compounds of general formula (Ia) may be prepared by the condensation of a salicylic aldehyde 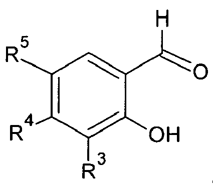 and a (Z) β-chloro-β-nitrostyrene 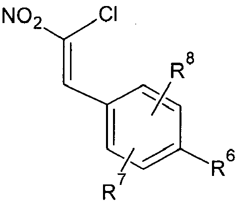 with triethylamine in THF in strictly anhydrous conditions.

More specifically,
- Compounds of Formula 3, 4, 5, 6, 7, 9, 11, 12, 13, 14, 15, 16, 19, 20, 21 and 25 may be prepared according to the article of D. DAUZONNE et al. (Synthesis, 66-70 (1990));
- Compounds of Formula 1, 17, 18, 22, 23, 24 and 28 may be prepared according to the article of D. DAUZONNE et al. (Eur. J. Med. Chem., 32, 71-82 (1997));
- Compounds of Formula 2 may be prepared according to the article of A. GONZALEZ DE PEREDO et al. (Chem. Pharm. Bull. 46, 79-83 (1998));
- Compounds of Formula 26, 27, 29 and 30 may be prepared according to the article of B. BAUVOIS et al. (J. Med. Chem. 46, 3900-3913 (2003)).

Another object of the present disclosure relates to compounds of Formula 8, 10, 31, 32, 33, 34, 35 and 36.

The hitherto unknown compounds 8, 10, 31, 32, 33, 34, 35 and 36 may be prepared, starting from the appropriate salicylaldehydes and (Z) b-chloro-b-nitrostyrenes, according to the methodology reported in the article of D. DAUZONNE et P. DEMERSEMAN (Synthesis, 66-70 (1990)).

According to the present disclosure preferred compounds of general formula (Ia) are compounds 5, 6, 7, 8, 9, 10, 11, 12, 13, 14, 15, 18, 20, 21, 22, 23, 24, 25, 27, 32 and 36.

More preferred compounds of general formula (Ia) are compounds 5, 6, 7, 8, 10, 11, 13, 15, 18, 21, 22, 23, 32 and 36.

The biological activities of the compounds of general formula (I) have been evaluated.

The ability of compounds of general formula (I) to restore respiratory growth of the yeast mutant has been evaluated with a yeast strain bearing a mutation on *ATP6* (Figure 3 and Example 1) and also with a yeast *fmc1Δ* mutant which affect the assembly of the ATPase complex.

These properties make said compounds as well as their salts suitable for use as drugs in the treatment of disorders or diseases linked with alteration of the F₁F₀-ATP synthase pathway; such diseases or disorders are those resulting from an insufficiency or a lack of ATP synthesis by F₁F₀-ATP synthase; and those related to an excessive accumulation of reactive oxygen species (ROS) which results from insufficiency or lack of ATP synthesis.

The use of the compounds of general formula (I) leads to an increase in ATP production and/or a decrease in ROS accumulation in cells.

As a consequence, compounds of general formula (I) are useful for the preparation of a drug for preventing and/or treating mitochondrial diseases, in particular for the treatment of mammals, such as human.

As already explained, mitochondrial diseases often result from a deficiency in ATP production -via the oxidative phosphorylation- which makes high energy-demanding tissues or organs such as heart, brain, and muscles, the main targets for these disorders.

Other mitochondrial alterations also contribute to pathologies amongst which an increased production of reactive oxygen species (ROS), responsible for important cellular oxidative damages, a low rate of NADH reoxidation and defective calcium storage within the organelle; examples of these pathologies are the syndromes NARP, LHON, MILS (*Maternally Inherited Leigh Syndrome*), MERRF (*Myoclonic Epilepsy with Ragged-Red Fibers*) and HSP (*Hereditary Spastic Paraplegia).*

Symptoms of mitochondrial diseases usually include slow growth, loss of muscle coordination, muscle weakness, visual defect, hearing defects, learning disabilities, mental retardation, heart disease, liver disease, kidney disease, gastrointestinal disorders, respiratory disorders, neurological problems, and dementia.

Compounds of general formula (I) appear to be also useful for the preparation of a drug for preventing and/or treating an acceleration of normal or physiological ageing. Physiological ageing is characterized by numerous phenomena; it may be characterized by the decrease of cells renewal; by the decrease of cells life and/or by the decrease of the number of cells in an organ, leading to atrophy and sometimes to dysfunction of said organ. Other evidences of ageing are modification of the appearance such as loss and/or graying of the hair, modification of appearance of the skin.

As mitochondrial disorders are observed during the progress of neurodegenerative diseases, the present invention also relates to the use of compounds of general formula (I) for preventing and/or treating neurodegenerative diseases such as Alzheimer's disease, myasteny disease, Parkinson's disease.

In another embodiment, the present disclosure relates to the use of compound of Formula C (Clotrimazole) 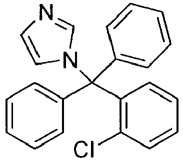 for the preparation of a drug for preventing and/or treating the symptoms of the mitochondrial diseases chosen in the group consisting of slow growth, muscle weakness, visual defect, hearing defect, heart disease, liver disease, kidney disease, gastrointestinal disorders and respiratory disorders, in particular for the treatment of mammals, such as human.

Clotrimazole is an antifungal agent.

This compound is commercially available; its synthese is reported in the literature: clotrimazole may be prepared according to South African Patents 68 05,392 and 69 00,039 (Bayer).

In addition to the above provisions, the disclosure also comprises other provisions which will become clear from the description which follows, which refers to examples illustrating the biological activity of compounds of general formula (I), and also to the attached drawings in which:
**Figure 1** is a schematic representation of the mitochondrial energy transduction apparatus and of the genes controlling the formation thereof.
**Figures 2A** **and** **2B** illustrate the structure of the ATP synthase and the genes encoding this protein complex: the ATP synthase is composed of subunits which are encoded by the nuclear DNA and by the mitochondrial DNA (Atp6p, Atp8p) and assembled in the mitochondrial matrix. Figure 2B focuses on the mitochondrial DNA and the mutations which are associated with human mitochondrial disease (from mitomap.org).
**Figures 3A and 3B** are pictures of Petri dishes showing the ability of compounds of general formula (I) to restore respiratory growth of the ATP synthase yeast mutant on nonfermentable medium. The three upper panels of figure 3A show that the ATP synthase mutants strains (three NARP strains and the *fmc1Δ*) grow very slowly on a respiratory medium (nonfermentable carbon source) due to a defect of the ATP synthase (Example 1).

The three lower panels of Figure 3A illustrate the improvement of the respiratory growth when compounds (Chlorhexidine, Benzethonium Chloride or Clotrimazole instead of DMSO, the negative control) are added into the agar medium at different concentrations.

On Figure 3B, the mutant cells are plated out in a layer at the surface of an agar medium containing nonfermentable carbon source and compounds are added on filters and disposed on Petri dish. The negative control, DMSO and the positive control, oleate, are loadded on the upper left filter and on the lower right filter respectively.

**Figures 4 to 8** are graphs showing the effect of compounds of general formula (I) on life expectancy of *Drosophila* having mitochondrial encephalomyopathy (Example 2).

**Figure 9** is a graph showing the effect of compound of Formula A (chlorhexidine, CH) on growth of a NARP mammalian model (Example 3).

### Example 1: Demonstration of the capacity of compounds of general formula (I) to restore respiratory growth of mutant yeasts on nonfermentable medium

The *ATP6* yeast mutants grow very slowly from a nonfermentable carbon source due to a dysfunction of the ATP synthase. These yeast mutants are therefore used to identify molecules capable of correcting the effects of the mutation by restoring either ATP synthase function, or ATP production by the mitochondria allowing the yeasts to grow on nonfermentable medium.

Mutant strains used in this experiment are:
- MC6 (*fmc1Δ*) (Lefebvre-Legendre L. et al., J. Biol. Chem., 2001, Mar2, 276 (9) : 6789-96)

Genotype: MC6: *Mat α, ade2, leu2, ura3, trpl, his3, fmc1::HIS3*
- MR14 (NARP T8993G) which may be obtained according to International PCT Application WO 2007/125225.

Genotype: MR14: *Mat a*, *ade2, leu2, ura3, trp1, his3, arg8::HIS3[rho⁺FY1679; atp6 T8993G]*.

The principle of the activity test is the following:
Step 1: the mutant yeast is cultured in medium containing glucose (YPAD medium).
Step 2: the mutant cells are plated out in a layer at the surface of an agar medium containing a nonfermentable carbon source such as glycerol (YPG medium).
Step 3: filters which each contain a defined amount of one of the test molecules are placed on the Petri dish, the molecules diffuse in the medium and establish a concentration gradient around the filters.
Step 4: the dishes are incubated at 35 or 36°C.

Under these conditions, a growth halo is seen to appear around the filters containing a substance capable of counteracting the effects of the mutation.

### Media

YPG medium contains 1% Yeast Extract; 2% Bactopeptone; 2% Bactoagar only for solid medium; 2% glycerol (expressed in weight/volume); Adenine 60mg/lL and spenicilline 30 000UI/L.

Liquid YPAD medium contains 1% Yeast Extract; 2% Bactopeptone; 2% glucose (expressed in weight/volume) and Adenine 60mg/L.

### The day before

For each of the MC6 and MR14 strains, one colony from a YPAD agar plate has been inoculated in 4 ml of liquid YPAD medium.

### The day of assay

In the morning, the optical density (OD) of the culture is measured; the culture is diluted in liquid YPAD medium (50 or 100 µl in 4ml of medium if the broth is saturated).

After 4-5 h, OD of the culture is measured; the culture is diluted in YPG medium until the OD is 0.2.

240 µl of the culture is introduced in squared dishes (12cm /12cm) and plated with sterile glass beads.

Sterile filters are introduced into the dishes.

Then filters are impregnated with either:
- 1 µl/filter of positive control (oleate 100 mM);
- 1 µl/filter of negative control (DMSO, compounds vehicle, pure from Sigma);
- 2.5 µl/filter of compounds having general formula (Ia) 10 mg/ml in DMSO;
- 2.5 µl/filter of compounds A, B, C and D 5 mg/ml in DMSO.

The dishes are incubated at 35°C for MR14 yeast strain or 36°C for MC6 yeast strain.

### Results

Results for chlorhexidine, benzethonium chloride or clotrimazole are illustrated in **Figures 3A****.**

Activity of the compounds is detected when mutant yeasts grow around the filters forming a growth halo; the size and the density of said halo allow defining a qualitative value for activity. All the selected drugs are active on both mutant strains (MR14 and *fmc1Δ*).

| **Compounds** | **activity** |
|---|---|
| 5 | ++ |
| 6 | ++ |
| 7 | ++ |
| 8 | +++ |
| 9 | + |
| 10 | ++ |
| 11 | ++ |
| 12 | + |
| 13 | ++ |
| 14 | + |
| 15 | ++ |
| 18 | ++ |
| 20 | + |
| 21 | ++ |
| 22 | ++ |
| 23 | ++ |
| 24 | + |
| 25 | + |
| 27 | + |
| 32 | ++ |
| 36 | ++ |
| A- Chlorhexidine | +++ |
| B- Benzethonium | ++ |
| C- Clotrimazole | +++ |
| D- Sodium Pyrithione * | ++ |

| | |
|---|---|
| * - Invention's example | |

### Example 2 - Demonstration of the capacity of compounds of general formula (I) to improve lifespan of Drosophila suffering from mitochondrial encephalomyopathy

Compounds of general formula (I) have then been tested on a *Drosophila* model described by Celotto et al. (Mitochondrial Encephalomyopathy in Drosophila, The Journal of Neuroscience, January 18, 2006 - 26(3):810-820). These Drosophila mutants are known to show a shorter lifespan.

### Methods for Drosophila Drug Screen

mtATP6[1];sesB[1]/sesB[1] flies were outcrossed once to produce females with the genotype mtATP6[1];sesB[1]/+ for study.

Each drug was dissolved in 0.09% DMSO to the following final concentrations: 15 µM, 1 µM, 50 nM and 2.5 nM.

Approximately 20 flies were tested per vial and 3 independent vials were tested for each concentration of each drug.

Newly eclosed females were counted and placed into a vial with approximately 10 milliliters standard cornmeal molasses media. Test compounds were applied (25 microliters at the specified concentrations) to a semi-circle of filter paper covering about 1/2 of the surface of the media.

Longevity experiments were performed using 12:12 light dark regime at 25 C.

Flies were counted every other day at which time food, filter paper and drug were replaced until all flies had expired. Survival curves were generated and analyzed using Prism 4.0b and log rank tests were performed to determine significance from the vehicle only controls.

### Results

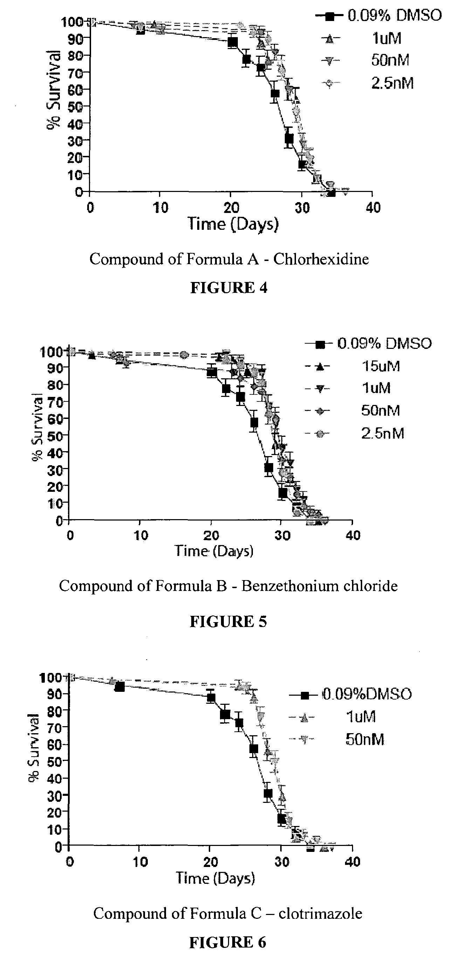

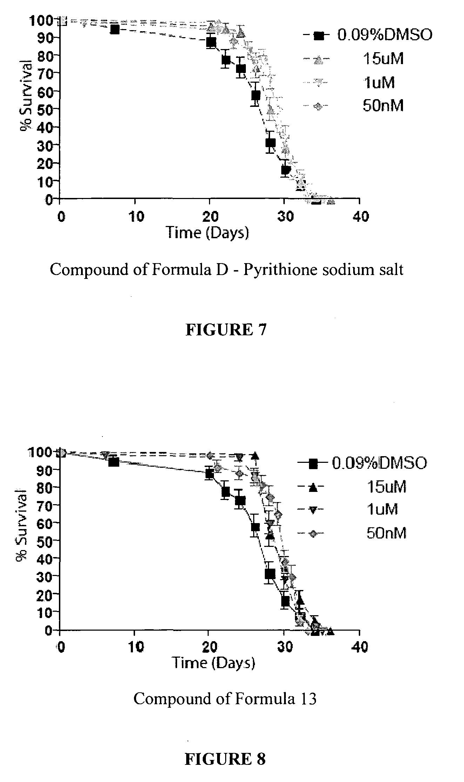

Graphs of Figures 4 to 8 show a significant increase in Drosophila's life after treatment with Compound of Formula A - Chlorhexidine (**Figure 4**), Compound of Formula B - Benzethonium chloride (**Figure 5**), Compound of Formula C - clotrimazole (**Figure 6**), Compound of Formula D - Pyrithione sodium salt (Invention's example) (**Figure 7**) and Compound of Formula 13 (**Figure 8**).

### Example 3 - Demonstration of the capacity of compound of Formula A (chlorhexidine) to improve the growth of a NARP mammalian model in medium deprivated of glucose (Reference example)

After the isolation of active drugs on the NARP yeast models, compounds' activity has been validated on mammalian models for the considered diseases.

Due to the limited number of cell divisions and the instability of heteroplasmy of mtDNA of the fibroblasts from the NARP patient, it has been decided to use the transmitochondrial cybrids, a cell line which is obtained by fusion of NARP patient's platelets containing heteroplasmic level of the mtDNA mutation with human osteosarcoma cells devoided of mtDNA. As fibroblasts, cybrids are using mainly a glycolytic metabolism. Therefore, in glucose medium, glycolysis provides the majority of the cellular ATP and both WT and NARP cybrids (JCP213 and JCP239 lines) present the same growth curves. On the contrary, in a medium supplemented with pyruvate and uridine but without glucose, the cells are forced to use a more oxidative metabolism (dependent on mitochondrial ATP production) (Weber, BioChem 2002).

The ability of JICP239 to grow in medium deprivated of glucose has been tested and used as a readout of the ability of the drugs selected in Example 1 to suppress NARP phenotype in human.

### - Cell lines and culture conditions

The cybrid lines JCP213 and JCP239 were generated by fusion of the human osteosarcoma cell line 143BK-ρ⁰ with platelets from wild-type patients or patients with T8993G mutation (Manfredi, G. et al. (1999). J Biol Chem 274, 9386-9391).

JCP213 contain 100% of WT mtDNA and JCP239 contain 84 ± 4% of mtDNA with T8993G transversion and were cultivated in Dulbecco's modified Eagle's medium (DMEM), high glucose (4.5 g.l⁻¹) supplemented with 5% fetal bovine serum (FBS Gold, PAA), 1 mM sodium pyruvate, 4 mM glutamine, 200 µM uridine and 20 U.ml⁻¹ penicillin/streptomycin at 37 °C in the atmosphere of 5% CO₂.

For growth rate measurements, 10⁴ cells were plated in a 24 well plates containing DMEM with glucose, as described above except the antibiotics. After 24 h, the growth medium is removed, the cells are washed with PBS and DMEM without glucose containing the drug or DMSO is added. For each condition of treatment, four wells were used.

Chlorhexidine (CH) solution in DMSO was diluted 1,000 times in the medium and used at final concentrations from 12.5 to 80 nM for CH.

DMSO and dihydrolipoic acid (DHLA) at 200 µM are respectively used as negative and positive controls.

After three days of incubation with the drugs, cell proliferation was estimated by Neutral Red staining (Aure, K. et al. (2007) Neuromuscul Disord 17, 368-37). Briefly, cells were incubated during 4 h at 37°C in presence of 33 µg.ml⁻¹ Neutral Red in DMEM without glucose, washed twice in PBS, and air-dried during 15 min. Neutral Red was then solubilized in 1 ml of 50% ethanol 1% acetic acid and quantified by its absorbance (540 nm).

Experiments were done at least three times per condition.

### - Measurement of the chlorhexidine activity

The NARP cybrids show a much slower growth than the WT cybrids. In order to evaluate the efficiency of the drugs isolated as active in the yeast-based screening, the above-described conditions of culture were used to test the effect of the drugs on the cell viability/proliferation of the NARP cybrids.

Hence, 24 hours after the seeding of the cybrids in 24 wells-plates in glucose medium, the medium was withdrawn, cells were washed and medium deprived of glucose (with pyruvate and uridine) and containing the tested compound was added. DMSO served as a negative control. After three days in presence of the drug, cell proliferation was estimated by Neutral Red staining (Aure *et al.* 2007). Each condition was performed using four wells and each experiment was done at least in triplicate. DMSO served as a negative control.

As for the yeast screening, the first step to validate this cybrid-based assay was to find a positive control.

The DHLA previously shown to partially correct the NARP fibroblasts deficiencies (Mattiazzi, M. et al. (2004) Hum Mol Genet 13, 869-879) and also uses in therapy to treat patients affected by mitochondrial neuropathies (DiMauro, S. et al. (2006). Muscle Nerve 34, 265-283) was tested as a positive control on the NARP cybrids growth.

After three days in a medium deprivated of glucose, the number of the NARP cybrids, JCP239, was increased by 2.2 fold in presence of 200 µM of DHLA. As for the yeast screening, the DHLA was used as a positive control.

Next CH has been tested; this compound has been shown active on the NARP yeast model using DHLA as a positive control: the day following the seeding, the glucose was removed from the medium and CH was added at concentrations ranging from 5 nM to 1 µM.

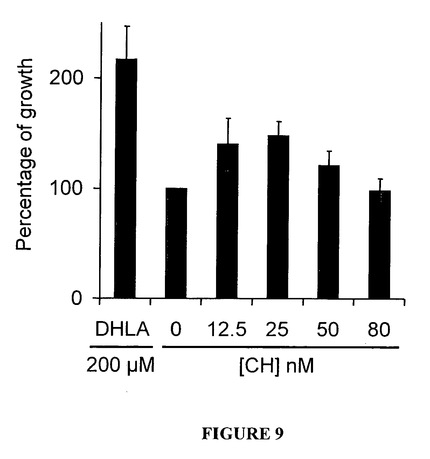

### - Results (Figure 9)

For concentrations ranging between 100 nM to 1 µM, CH was toxic and induced the cells death. In contrast, at concentrations comprised between 12.5 nM and 50 nM, NARP cybrids growth was clearly improved. In presence of 12.5, 25 and 50 nM of CH, the NARP cybrids growth was increased by 1.4, 1.5 and 1.2 respectively (see **Figure 9**).

Therefore, the treatment with DHLA or CH improved NARP cybrids growth in condition where oxidative metabolism was necessary.

## Claims

1. Sodium pyrithione for its use in preventing and/or the treating mitochondrial diseases and/or mitochondrial alterations and/or mitochondrial disorders observed during the progress of neurodegenerative diseases.

2. Sodium pyrithione for use as claimed in claim 1, wherein mitochondrial alterations are selected in the group consisting of the syndromes NARP (*Neuropathy, Ataxia and Retinitis Pigmentosa*), LHON (*Leber*'*s Hereditary Optic Neuropathy),* MILS (*Maternally Inherited Leigh Syndrome*), MERRF (*Myoclonic Epilepsy with Ragged-Red Fibers*) and HSP *(Hereditary Spastic Paraplegia).*

3. Sodium pyrithione for use as claimed in claim 1, wherein the symptoms of said mitochondrial diseases are selected in the group consisting of slow growth, muscle weakness, visual defect, hearing defect, heart disease, liver disease, kidney disease, gastrointestinal disorders and respiratory disorders.

## Patentansprüche

1. Natriumpyrithion für seine Verwendung bei der Prävention und/oder der Behandlung von mitochondrialen Krankheiten und/oder von mitochondrialen Veränderungen und/oder von mitochondrialen Störungen, die während des Fortschreitens von neurodegenerativen Erkrankungen beobachtet werden.

2. Natriumpyrithion zur Verwendung nach Anspruch 1, wobei mitochondriale Veränderungen in der Gruppe ausgewählt werden, die aus den Syndromen NARP (*Neuropathy, Ataxia and Retinitis Pigmentosa*)*,* LHON (*Leber*'*s Hereditary Optic Neuropathy*), MILS (*Maternally Inherited Leigh Syndrome*), MERRF (*Myoclonic Epilepsy with Ragged-Red Fibers*) und HSP (*Hereditary Spastic Paraplegia*) besteht.

3. Natriumpyrithion zur Verwendung nach Anspruch 1, wobei die Symptome der mitochondrialen Erkrankungen in der Gruppe ausgewählt werden, die aus langsamem Wachstum, Muskelschwäche, Sehfehler, Hörfehler, Herzerkrankung, Lebererkrankung, Nierenerkrankung, gastrointestinalen Beschwerden und Atembeschwerden besteht.

## Revendications

1. Pyrithione de sodium pour son utilisation dans la prévention et/ou le traitement de maladies mitochondriales et/ou de modifications mitochondriales et/ou de troubles mitochondriaux observés durant la progression de maladies neurodégénératives.

2. Pyrithione de sodium pour son utilisation selon la revendication 1, dans laquelle les modifications mitochondriales sont sélectionnées dans le groupe constitué des syndromes NARP (neuropathie, ataxie, et rétinite pigmentaire), LHON (neuropathie optique héréditaire de Leber), MILS (syndrome de Leigh de transmission maternelle), MERRF (épilepsie myoclonique avec fibres rouges déchiquetées) et HSP (paraplégie spastique héréditaire).

3. Pyrithione de sodium pour son utilisation selon la revendication 1, dans laquelle les symptômes desdites maladies mitochondriales sont sélectionnés dans le groupe constitué d'une croissance lente, d'une faiblesse musculaire, d'un déficit visuel, d'un déficit auditif, d'une maladie cardiaque, d'une maladie hépatique, d'une maladie rénale, de troubles gastro-intestinaux, et de troubles respiratoires.
